# EUROPÄISCHE PATENTSCHRIFT

(11) **EP 1 317 672 B1**
(45) Veröffentlichungstag und Bekanntmachung des Hinweises auf die Patenterteilung: **23.03.2005**
(21) Anmeldenummer: 01974230.3
(22) Anmeldetag: 06.09.2001
(51) Int. Cl.: G01N 33/86

(54) **VERFAHREN ZUR MESSUNG DER AKTIVITÄT VON GERINNUNGSFAKTOR XIIIa**
METHOD FOR MEASURING THE ACTIVITY OF THE BLOOD CLOTTING FACTOR XIIIA
METHODE DE MESURE DE L'ACTIVITE DU FACTEUR DE COAGULATION XIIIA

(30) Priorität: 13.09.2000 DE 10046187
(43) Veröffentlichungstag der Anmeldung: 11.06.2003
(73) Patentinhaber: Friedrich-Schiller-Universität Jena, 07743 Jena (DE)
(72) Erfinder: PRASA, Dagmar, 99094 Erfurt (DE); STÜRZEBECHER, Jörg, 99094 Erfurt (DE)
(74) Vertreter: Bock, Gerhard, Dipl.-Ing.
(86) Internationale Anmeldenummer: PCT/EP2001/010285
(87) Internationale Veröffentlichungsnummer: WO 2002/022853

(56) Entgegenhaltungen:
- WO-A-98/58078
- COYNE C P ET AL: "PHARMACOLOGIC EVALUATION OF FACTOR XIIIA*-LIKE ENZYME ACTIVITY IN EQUINE PLASMA AS A POTENTIAL THERAPEUTIC AVENUE FOR THE INHIBITION OF FIBRINOUS TISSUE" AMERICAN JOURNAL OF VETERINARY RESEARCH, XX, XX, Bd. 53, Nr. 5, 1. Mai 1992 (1992-05-01), Seiten 695-705, XP000195966 ISSN: 0002-9645
- RETZINGER G S ET AL: "QUANTITIATION OF PLASMA FACTOR XIIIA ACTIVITY USING FIBRIN-COATED MICROSCOPIC LATEX BEADS" ANALYTICAL BIOCHEMISTRY, Bd. 195, Nr. 1, 1991, Seiten 18-23, XP002204879 ISSN: 0003-2697
- PRASA D ET AL: "A screening method for inhibitors of factor XIIIa." ANNALS OF HEMATOLOGY, Bd. 80, Nr. Supplement 1, 2001, Seite A54 XP008005518 45th Annual Meeting of the Society for Thrombosis/Hemostasis Research;Duesseldorf, Germany; February 14-17, 2001 ISSN: 0939-5555

## Beschreibung

Die Erfindung betrifft ein Verfahren zur Messung der Aktivität von Gerinnungsfaktor XIIIa, insbesondere zum Einsatz von Testsystemen mit hohem Durchsatz.

Der Gerinnungsfaktor XIIIa (FXIIIa) ist als Transglutaminase das einzige nicht proteolytisch wirkende Enzym im plasmatischen Gerinnungssystem. Das im Plasma zirkulierende inaktive Proenzym Faktor XIII (FXIII) wird im Verlauf der Blutgerinnung durch Thrombin aktiviert und katalysiert die intermolekulare Vernetzung von Fibrinmonomeren untereinander und mit anderen Plasmaproteinen (z. B. α₂-Antiplasmin) durch Bildung von ε-(-γ-Glutamyl)-Lysin-Bindungen. Dadurch werden die mechanische Festigkeit und die Widerstandsfähigkeit des Fibringerinnsels gegenüber fibrinolytischem Abbau verstärkt.

Außerhalb des Gerinnungsgeschehens spielt FXIIIa eine Rolle bei der Wundheilung. FXIII wurde auch in verschiedenen Zellen (Thrombozyten, Makrophagen) und Geweben (Plazenta) nachgewiesen. Über die Funktion(en) des zellulären FXIIIa ist bisher wenig bekannt (L. Muszbek et al.: Thromb. Res. 94, 1999, 271-305).

Für die weitere Aufklärung der physiologischen Rolle von FXIIIa fehlen allerdings wirksame, spezifische Inhibitoren. Für derartige Hemmstoffe wird eine therapeutische Anwendung zur Thromboseprophylaxe diskutiert, die Kombination mit Plasminogenaktivatoren fördert die Lyse von thrombotischen Verschlüssen (E. M. Leidy et al.: Thromb. Res. 59, 1990, 15-26; R. J. Shebuski et al.: Blood 75, 2000, 1455-1459).

Bisher wurden nur wenige FXIIIa-Hemmstoffe beschrieben. In der Mehrzahl handelt es sich um Substrate vom Typ substituierter Alkylamine, die mit den Glutamin-Resten im Fibrin verknüpft werden und damit eine Quervernetzung des Fibrins verhindern. Um eine ausreichende Wirkung zu erzielen, sind allerdings hohe Konzentrationen dieser Pseudosubstrate erforderlich (S.-Y. Kim et al.: Biochem. Biophys. Res. Commun. 233, 1997, 39-44).

Als direkte Hemmstoffe von FXIIIa wurden u. a. Imidazolderivate, Triazol- und Tetrazoliumverbindungen beschrieben, welche die Sulfhydrylgruppe im aktiven Zentrum des Enzyms irreversibel blockieren (US 5,077,285; US 5,177,092; US 5,047,416).

Der von der Firma Merck Sharp & Dohme entwickelte FXIIIa-Hemmstoff L722151, ein Thiazolo-thiadiazolium-Derivat, ist in der Lage, im Tiermodell eine Verbesserung der Thrombolyse mit einem Plasminogenaktivator zu erreichen (E. M. Leidy et al.: Thromb. Res. 59, 1990, 15-26).

Neben den synthetischen Hemmstoffen sind auch natürliche Hemmstoffe von FXIIIa bekannt. Das hochwirksame Tridegin wurde aus dem Egel Haementeria ghilianii, andere Hemmstoffe aus verschiedenen Mikroorganismen isoliert (US 6,025,330; US 5,710,174; K. Ikura et al.: Biosci. Biotechnol. Biochem. 64, 2000, 116-124).

Den bekannten Methoden zur Aktivitätsbestimmung von FXIIIa liegen zwei Prinzipien zu Grunde:
(1) der Clot-Solubility-Test nutzt die unterschiedliche Löslichkeit von vernetzten und nicht vemetzten Fibringerinnseln in Harnstoff, erlaubt aber nur eine halbquantitative Bestimmung der FXIIIa-Aktivität (P. Sigg: Thromb. Diath. Haemorrh. 15, 1966, 238-251; A. A. Tymiak et al.: J. Antibiotics 46, 1993, 204-206).
(2) wird die katalytische Eigenschaft von FXIIIa genutzt, um markierte oder biotinylierte Aminsubstrate (Dansylcadaverin, Biotinpentylamin) an gewöhnlich immobilisierte Peptide (z. B. Casein oder Fibrin) zu binden. Die Bestimmung des gebundenen Substrates erfolgt direkt (Änderungen der Fluoreszenz bzw. Chemilumineszenz) oder über Antikörper- bzw. Streptavidin-gekoppelte Enzyme (US 5,015,588; US 4,601,977). Alle diese Verfahren erfordern mehrfache Inkubationsschritte, sind daher zeit- und arbeitsaufwendig und für die Suche nach neuen, wirksamen und spezifischen Hemmstoffen von FXBIa nicht geeignet, da sie die Anwendung von automatisierten Testsystemen mit hohem Durchsatz nicht ermöglichen. Auch die Methode, bei der Glycinethylester mit einem glutaminhaltigen Peptidsubstrat verknüpft wird und der dabei freigesetzte Ammoniak in einer enzymatischen Reaktion gemessen wird, eignet sich nicht für ein Screening mit hohem Durchsatz (K. Fickenscher et al.: Thromb. Haemost. 65, 1991, 535-540).

Der Erfindung liegt die Aufgabe zu Grunde, eine leicht durchführbare Methode zur Messung der Aktivität von FXIIIa zu beschreiben, die insbesondere für das Screening nach FXIIIa-Hemmstoffen unter Einsatz von Testsystemen mit hohem Durchsatz angewendet werden kann.

Überraschend wurde gefunden, dass FXIIIa in Abhängigkeit von der eingesetzten Konzentration die Lichtdurchlässigkeit von Fibringerinnseln - entstanden durch die Wirkung eines Fibrinogen-spaltenden Enzyms wie z. B. Thrombin oder Batroxobin - verändert.

Auf der Grundlage dieser Ergebnisse wurde eine Methode zur Messung der Aktivität von FXIIIa entwickelt, die im Folgenden näher beschrieben wird.

Fibrinogen wird mit einem Fibrinogen-spaltenden Enzym zur Gerinnung gebracht. Vorzugsweise wird das Schlangengiftenzym Batroxobin eingesetzt, ein thrombinartiges Enzym aus dem Gift von Bothrops atrox, das im Gegensatz zu Thrombin nur Fibrizlopeptid A aus dem Fibrinogenmolekül abspaltet. Der Einsatz von Thrombin ist ebenfalls möglich. Als besonders günstig erweist sich die Verwendung von gereinigtem Fibrinogen vom Menschen oder einem anderen Säuger. Als Fibrinogenquelle kann aber auch antikoaguliertes Plasma (z. B. durch Citrat oder Hirudin) vom Menschen oder von einem anderen Säuger genutzt werden. Vorzugsweise wird das erfindungsgemäße Verfahren in einer Pufferlösung durchgeführt. Bevorzugt wird Tris-HCl-Puffer mit einem pH-Wert von 7,4. Um die Sensitivität des Testverfahrens zu erhöhen, wird dem Messansatz eine Substanz - gewöhnlich ein Polymer - zugesetzt, die in der Lage ist, die Struktur der entstehenden Fibrinfasern derart zu verändern, dass die Lichtdurchlässigkeit des Gerinnsels vermindert wird. Vorzugsweise wird Polyethylenglykol 6000 in einer Konzentration von 0,2 % eingesetzt.

Die durch die Fibrinogengerinnung auftretende Extinktionszunahme (Trübung) wird kontinuierlich über einen bestimmten Zeitraum photometrisch erfasst. Eine Endpunktmessung ist ebenfalls möglich. Die Messung wird bevorzugt bei 37°C und einer Wellenlänge von 405 nm durchgeführt. Wird dem Messansatz aktivierter FXIII zugesetzt, ist die Extinktionszmahme während der Reaktion geringer, d. h. die Lichtdurchlässigkeit des entstandenen Gerinnsels ist größer. Die Differenz zwischen der Extinktionsänderung bzw. der Fläche unter der Extinktions-Zeit-Kurve von einem Ansatz ohne FXIIIa und einem Ansatz mit FXIIIa dient als Maß für die Aktivität von FXIIIa. Mit dem beschriebenen Testansatz können Hemmstoffe von FXIIIa aufgefunden werden, da ein Hemmstoff die Lichtdurchlässigkeit des Gerinnsels entsprechend der Stärke der Hemmwirkung erniedrigt. Die Aktivität von Faktor XIIIa mit und ohne Testsubstanz wird verglichen. Die Differenz der Aktivität dient als Maß für die Hemmwirkung der untersuchten Testsubstanz. Bei der Verwendung von Thrombin als fibrinogenspaltendes Enzym kann parallel nach Thrombinhemmstoffen gesucht werden. Besitzen die zu untersuchenden Substanzen eine Hemmwirkung gegenüber Thrombin, wird die Fibrinogengerinnung verhindert, die Extinktion der Probe verändert sich nicht.

Das erfindungsgemäße Verfahren kann sowohl zur Messung der Aktivität von plasmatischem als auch zellulärem FXIIIa eingesetzt werden. Das Proenzym FXIII wird vor der Messung in einem gesonderten Ansatz aktiviert. Die Aktivierung erfolgt durch Thrombin in Gegenwart von Ca-Ionen. Um bei der Verwendung anderer fibrinogenspaltender Enzyme wie Batroxobin den störenden Einfluss des Thrombins auf die Messung zu verhindern, wird nach der vollständigen Aktivierung des FXIII dem Aktivierungsansatz ein natürlicher oder synthetischer Hemmstoff des Thrombins in ausreichender Konzentration zugesetzt. Vorzugsweise wird rekombinantes Hirudin verwendet. Beim Einsatz von Thrombin als fibrinogenspaltendes Enzym entfällt dieser Schritt.

Die Erfindung soll nachstehend anhand von vier in der Zeichnung dargestellten Ausführungsbeispielen näher erläutert werden.

Es zeigen:
- Fig. 1:: Gerinnung von Citrat-antikoaguliertem Plasma mit Batroxobin in Gegenwart von aktiviertem FXIII
- Fig. 2:: Einfluss verschiedener Polymere auf die Gerinnung von Fibrinogen mit Batroxobin
- Fig. 3:: Gerinnung von Fibrinogen durch Batroxobin in Gegenwart verschiedener FXIIIa-Konzentrationen
- Fig. 4:: Hemmung von FXIIIa durch Jodessigsäure
Fig. 4A: Messkurve
Fig. 4B: FXIIIa-Aktivität in Abhängigkeit von der Hemmstoffkonzentration

### Ausführungsbeispiel 1:

### Gerinnung von Citrat-antikoaguliertem Plasma mit Batroxobin in Gegenwart von aktiviertem FXIII

### Aktivierung von FXIII:

865 µl Tris-Puffer (0,05 M; 0,154 M NaCl; pH 7,4), 25 µl CaCl₂ (0,2 M), 100 µl Thrombin (aus Rinderplasma, 100 E/ml) und 10 µl zellulärer rekombinanter FXIII (Aventis-Behring, Marburg, Deutschland; 8,6 mg/ml bzw. 1155 E/ml) werden gemischt und 10 min bei 37 °C inkubiert. Anschließend werden 25 µl rekombinantes Hirudin (HBW 023, Höchst, Frankfurt, Deutschland; 1000 E/ml) zugegeben.

### Messansatz:

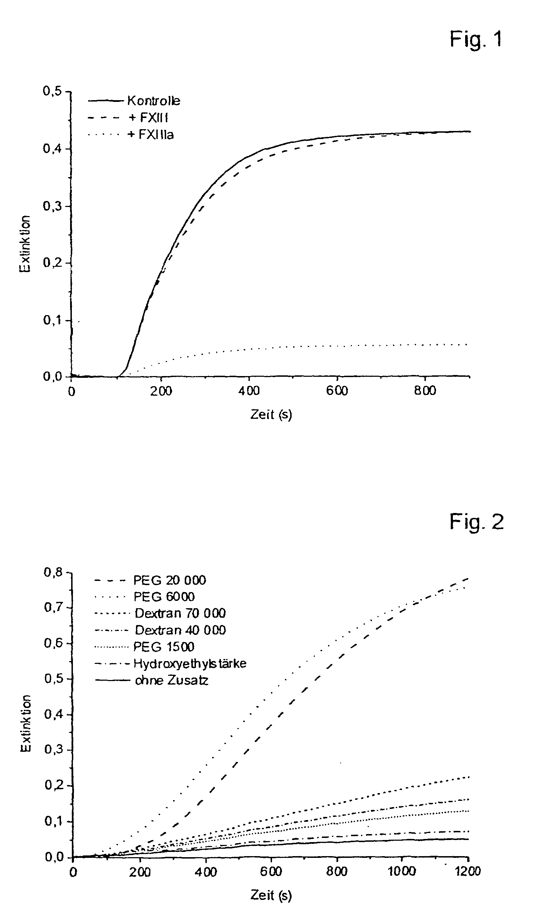

Auf einer Mikrotiterplatte werden 100 µl Citrat-Plasma, 60 µl Tris-Puffer (pH 7,4) und 40 µl des oben aufgeführten Aktivierungsansatzes gemischt und auf 37 °C temperiert. Die Reaktion wird durch Zugabe von 50 µl Batroxobin (Pentapharm Ltd., Basel, Schweiz; 2,5 E/ml) gestartet. Die Extinktionszunahme wird über 15 min bei 37 °C und einer Wellenlänge von 405 nm in einem Mikrotiterplattenphotometer (iEMS, Labsystems, Helsinki, Finnland) verfolgt. Ein Messansatz ohne Faktor XIIIa dient als Kontrolle.
Die Messkurven sind in Fig. 1 dargestellt.

### Ergebnis:

Nach einer kurzen Lagphase nimmt mit dem Einsetzen der Gerinnung die Extinktion zu und nähert sich im hinteren Teil der Messkurve einem Plateauwert. In Gegenwart von aktiviertem FXIII ist die Extinktionszunahme deutlich geringer als in Proben ohne FXIIIa. Nichtaktivierter FXIII hat keinen Einfluss auf die Lichtduchlässigkeit des Gerinnsels.

### Ausführungsbeispiel 2:

### Einfluss verschiedener Polymere auf die Gerinnung von Fibrinogen mit Batroxobin

100 µl Fibrinogen (Sigma, Deisenhofen, Deutschland; 0,6%); 25 µl eines Polymers (25 mg/ml); 75 µl Tris-Puffer werden auf einer Mikrotiterplatte gemischt und auf 37 °C temperiert. Nach Zugabe von 50 µl Batroxobin (2,5 E/ml) wird die Extinktionszunahme über 20 min unter den in Ausführungsbeispiel 1 aufgeführten Bedingungen gemessen.
Der Einfluss von Hydroxyethylstärke, Dextransulfat und Polyethylenglykol auf die Lichtdurchlässigkeit von Fibrinogengerinnseln wird in Fig. 2 gezeigt.

### Ergebnis:

Die zugesetzten Polymere bewirken eine verminderte Lichtdurchlässigkeit der Gerinnsel, sichtbar in einer verstärkten Zunahme der Extinktion. Der Einfluss von Hydroxyethylstärke, Dextran und Polyethylenglykol 1500 ist relativ gering. Polyethylenglykol 6000 und 20000 dagegen erhöhen die Extinktion um ca. das 15-fache im Vergleich zu Proben ohne Polymerzusatz.

### Ausführungsbeispiel 3:

### Gerinnung von Fibrinogen durch Batroxobin in Gegenwart verschiedener FXIIIa-Konzentrationen

Die Aktivierung von FXIII erfolgt gemäß Ausführungsbeispiel 1.

Auf einer Mikrotiterplatte werden 100 µl Fibrinogen (0,6%), 25 µl PEG 6000 (2 %), 100 µl Aktivierungsansatz (mit verschiedenen Konzentrationen von FXIIIa) gemischt und nach dem Temperieren auf 37 °C mit 25 µl Batroxobin (5 E/ml) versetzt. Die Extinktion wird wie in Ausführungsbeispiel 2 gemessen. Die Extinktionszunahme der Proben wird in % bezogen auf die Extinktionszunahme in einer Probe ohne FXIIIa (Kontrolle = 100%) angegeben.

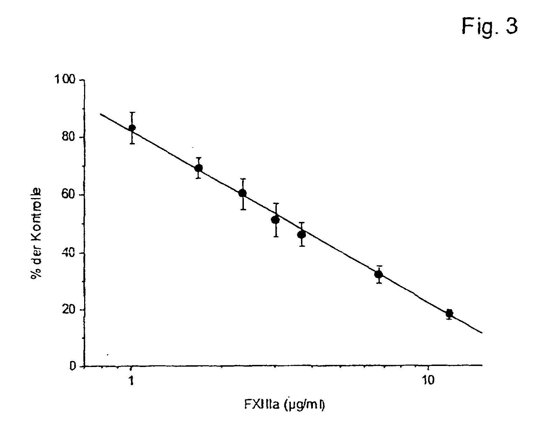

Fig. 3 zeigt die Eichkurve für FXIIIa. Dargestellt sind die Mittelwerte (n = 6) ± Standardabweichung.

### Ergebnis:

FXIIIa erhöht konzentrationsabhängig die Lichtdurchlässigkeit der Gerinnsel. Die Eichkurve verläuft linear bei FXIIIa-Konzentrationen zwischen 1 und 12 µg/ml.

### Ausführungsbeispiel 4:

### Hemmung von FXIIIa durch Jodessigsäure

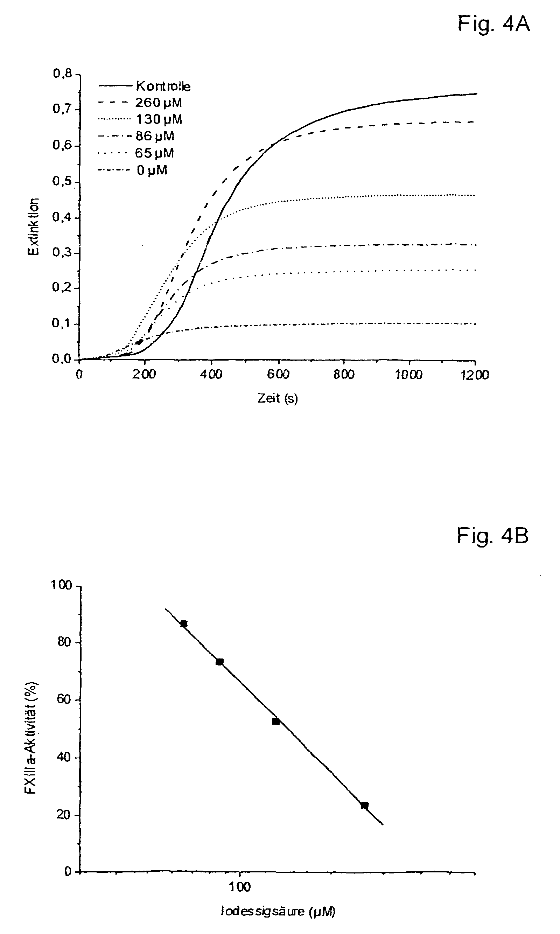

Die Aktivierung von FXIII erfolgt gemäß Ausführungsbeispiel 1.
100 µl Fibrinogen (0,6 %), 25 µl PEG 6000 (2 %), 80 µl Jodessigsäure in Puffer (0,2 - 0,8 mM) und 20 µl Aktivierungsansatz werden gemischt und auf 37 °C temperiert. Nach Zugabe von 25 µl Batroxobin (5 E/ml) wird die Extinktion wie oben beschrieben gemessen.
Die Messkurven sind in Fig. 4A dargestellt. Figur 4B zeigt die FXIIIa-Aktivität in Abhängigkeit von der Hemmstoffkonzentration.

### Ergebnis:

Jodessigsäure hemmt konzentrationsabhängig die Aktivität von FXIIIa, erkennbar an einer verminderten Lichtdurchlässigkeit des Gerinnsels. Als Maß für die Hemmwirkung wird die Hemmstoffkonzentration (IC₅₀) ermittelt, die eine Reduzierung der FXIII-Aktivität um 50% bewirkt. Der IC₅₀-Wert für Jodessigsäure beträgt 147 µM.

## Patentansprüche

1. Verfahren zur Messung der Aktivität von Gerinnungsfaktor XIIIa in einer Probenlösung, welche Faktor XIIIa enthält oder in welcher FXIIIa entsteht, **dadurch gekennzeichnet, dass** der genannten Probenlösung Fibrinogen zugesetzt wird, welches mit einem fibrinogenspaltenden Enzym zur Gerinnung gebracht wird, und die dabei auftretende Extinktionsänderung gemessen und ausgewertet wird.

2. Verfahren nach Anspruch 1, **dadurch gekennzeichnet, dass** als fibrinogenspaltendes Enzym Batroxobin verwendet wird.

3. Verfahren nach Anspruch 1, **dadurch gekennzeichnet, dass** als fibrinogenspaltendes Enzym Thrombin verwendet wird.

4. Verfahren nach Ansprüchen 1 und 2, **dadurch gekennzeichnet, dass** als Fibrinogenquelle antikoaguliertes Plasma benutzt wird.

5. Verfahren nach Ansprüchen 1 bis 3, **dadurch gekennzeichnet, dass** dem Ansatz zur Extinktionsmessung eine Substanz, insbesondere ein Polymer, zugesetzt wird, welche die Lichtdurchlässigkeit des Gerinnsels vermindert.

6. Verfahren nach Anspruch 5, **dadurch gekennzeichnet dass** als Polymer Polyethylenglykol 6000 eingesetzt wird.

7. Verwendung des Verfahrens gemäß Ansprüchen 1 bis 6 zum Screening nach Hemmstoffen von FXIIIa.

8. Verwendung des Verfahrens gemäß Anspruch 3 zum gleichzeitigen Screening sowohl nach Hemmstoffen von FXIIIa als auch von Thrombin.

## Claims

1. A method for measuring the activity of the clotting factor XIIIa in a sample solution which contains factor XIIIa or in which FXIIIa is formed, wherein fibrinogen is added to said sample solution and caused to coagulate by a fibrinogen-splitting enzyme, and the resulting change in extinction is measured and evaluated.

2. The method according to Claim 1, wherein batroxobin is used as the fibrinogen-splitting enzyme.

3. The method according to Claim 1, wherein thrombin is used as the fibrinogen-splitting enzyme.

4. The method according to Claims 1 and 2, wherein anticoagulated plasma is used as the source of fibrinogen.

5. The method according to Claims 1 through 3, wherein a substance, a polymer in particular, is added to the preparation solution used for measuring the extinction and which reduces the light transmitting power of the clot.

6. The method according to Claim 5, wherein polyethyleneglycol 6000 is used as the polymer.

7. The application of the present method according to Claims 1 through 6 for screening inhibitors of FXIIIa.

8. The application of the present method according to Claim 3 for the simultaneous screening both of inhibitors of FXIIIa and thrombin.

## Revendications

1. Méthode de mesure de l'activité du facteur de coagulation XIIIa dans une solution d'agents qui comprend le facteur XIIIa ou dans laquelle se forme le facteur FXIIIa, est **caractérisée en ce qu'**à la solution en question est additionné du fibrinogène qui coagule sous l'action d'un enzyme possédant la capacité de scinder le fibrinogène et que les changements d'extinction survenus au cours de ce processus sont mesurés et évalués.

2. Le procédé selon la revendication 1 est **caractérisé en ce que** l'enzyme utilisé et possédant la capacité de scinder le fibrinogène, est la batroxobine.

3. Le procédé selon la revendication 1 est **caractérisé en ce que** l'enzyme utilisé possédant la capacité de scinder le fibrinogène, est la thrombine.

4. Le procédé selon les revendications 1 et 2 est **caractérisé en ce que** le fibrinogène provient de plasma anticoagulant.

5. Le procédé selon les revendications 1 à 3 est **caractérisé en ce qu'**il faut additionner à la solution préparée destinée à la mesure de l'extinction, une substance, de préférence un polymère, réduisant la transparence du caillot.

6. Le procédé selon la revendication 5 est **caractérisé en ce que** le polymère utilisé à cet effet est le polyéthylène glycol 6000.

7. Application du procédé suivant les revendications 1 à 6 pour effectuer un screening par agents inhibiteurs de FXIIIa.

8. Application du procédé suivant la revendication 3 pour effectuer le screening simultané par agents inhibiteurs de FXIIIa et de Thrombine.
